# EUROPEAN PATENT APPLICATION

(11) **EP 4 552 688 A2**
(43) Date of publication of application: **14.05.2025**
(21) Application number: 24209704.6
(22) Date of filing: 30.10.2024
(51) Int. Cl.: A61N 5/06, A61B 5/24, A61N 1/18

(54) **PHOTOBIOMODULATION SYSTEMS UTILIZING MONITORING OR ELECTRICAL STIMULATION AND METHODS OF MAKING AND USING**

(30) Priority: 09.11.2023 US 202363547925 P
(71) Applicant: Boston Scientific Neuromodulation Corporation, Valencia, CA 91355 (US)
(72) Inventor: MOFFITT, Michael A., Valencia, CA, 91355 (US); SUBRAMANIAN, Hari Hara, Valencia, CA, 91355 (US); CARBUNARU, Rafael, Valencia, CA, 91355 (US)
(74) Representative: Vossius & Partner Patentanwälte Rechtsanwälte mbB

(57) **Abstract**

A method for photobiomodulation includes emitting light from a lead implanted in a patient using a light source according to a first delivery program to photobiomodulate the patient; monitoring at least one biomarker, an evoked signal, or a local field potential to monitor at least one effect of the photobiomodulation; determining, by a processor, whether the monitoring of the at least one biomarker meets a change condition; when the monitoring of the at least one biomarker meets a change condition, determining, by the processor, a change to the first delivery program to produce a second delivery program; and emitting light from the implanted lead according to the second delivery program. Other aspects include coordinated photobiomodulation and electrical stimulation.

## Description

### FIELD

The present disclosure is directed to the area of implantable photobiomodulation (PBM) or PBM/electrical stimulation systems and methods of making and using the systems. The present disclosure is also directed to implantable PBM or PBM/electrical stimulation systems utilize monitoring of one or more biomarkers.

### BACKGROUND

Implantable neuromodulation systems have proven therapeutic in a variety of diseases and disorders. For example, spinal cord stimulation systems have been used as a therapeutic modality for the treatment of chronic pain syndromes. Peripheral nerve stimulation has been used to treat chronic pain syndrome and incontinence. Functional electrical stimulation systems have been applied to restore some functionality to paralyzed extremities in spinal cord injury patients. Stimulation of the brain, such as deep brain stimulation, can be used to treat a variety of diseases or disorders.

Stimulators have been developed to provide therapy for a variety of treatments. A stimulator can include a control module (with a pulse generator), one or more leads, and an array of stimulator electrodes on each lead. The stimulator electrodes are in contact with or near the nerves, muscles, or other tissue to be stimulated. The pulse generator in the control module generates electrical pulses that are delivered by the electrodes to body tissue.

Photobiomodulation (PBM) can also provide therapeutic benefits in a variety of diseases and disorders by itself or in combination with electrical stimulation. An PBM system may include one or more light sources and, often, one or more optical fibers to carry the light to the desired modulation site.

### BRIEF SUMMARY

One aspect is a method for photobiomodulation. The method includes emitting light from a lead implanted in a patient using a light source according to a first delivery program to photobiomodulate the patient; monitoring at least one biomarker to monitor at least one effect of the photobiomodulation; determining, by a processor, whether the monitoring of the at least one biomarker meets a change condition; when the monitoring of the at least one biomarker meets a change condition, determining, by the processor, a change to the first delivery program to produce a second delivery program; and emitting light from the implanted lead according to the second delivery program.

Another aspect is a photobiomodulation system that includes a lead implantable in a patient; a light source; a control module coupled to, or containing, the lead and the light source and configured for directing emission of light from the lead using the light source according to a first delivery program to photobiomodulate the patient; at least one sensor configured to monitor at least one biomarker to monitor at least one effect of the photobiomodulation; and a processor configured for determining whether the monitoring of the at least one biomarker meets a change condition; and when the monitoring of the at least one biomarker meets a change condition, determining a change to the first delivery program to produce a second delivery program for emission of the light from the lead according to the second delivery program.

In at least some aspects, the change condition includes a magnitude or a rate of the biomarker. In at least some aspects, the change condition includes a magnitude or rate of a change in a magnitude of the biomarker.

In at least some aspects, the monitoring includes monitoring the at least one biomarker using at least one sensor. In at least some aspects, at least one of the at least one sensor is an implanted sensor. In at least some aspects, at least one of the at least one sensor is a worn or wearable sensor.

In at least some aspects, the monitoring or the determining is performed at a regular periodic interval. In at least some aspects, the biomarker includes dopamine or acetylcholine. In at least some aspects, the emitting includes emitting the light for neural protection, neural survival, or neural growth.

Yet another aspect is a method for photobiomodulation of tissue of a brain of a patient. The method includes emitting light at a first site in the brain from an implanted lead using a light source according to a first delivery program to photobiomodulate the tissue; in response to the emitting, monitoring an evoked signal or local field potential at a second site in the brain that is spaced apart from the first site by at least 5 mm; determining whether the monitoring of the evoked signal or local field potential meets a change condition; when the monitoring of the evoked signal or local field potential meets a change condition, determining a change to the first delivery program to produce a second delivery program; and emitting light from the implanted lead according to the second delivery program.

A further aspect is a photobiomodulation system that includes a lead implantable in a patient; a light source; a control module coupled to, or containing, the lead and the light source and configured for directing emission of light from the lead at a first site using the light source according to a first delivery program to photobiomodulate the patient; a sensor configured to monitor an evoked signal or local field potential at a second site; and a processor configured for monitoring the evoked signal or local field potential at the second site in response to the emission of light; determining, by a processor, whether the monitoring of the evoked signal or local field potential meets a change condition; and when the monitoring of the evoked signal or local field potential meets a change condition, determining a change to the first delivery program to produce a second delivery program for emission of the light from the lead according to the second delivery program.

In at least some aspects, the change condition includes a magnitude or change in a magnitude of the evoked signal. In at least some aspects, the change condition includes a magnitude or a change in magnitude of a local field potential.

In at least some aspects, the monitoring or the determining is performed at a regular periodic interval. In at least some aspects, the determining includes determining whether the monitoring of the evoked signal or local field potential meets the change condition accounting for an additional factor selected from a circadian rhythm or a current activity of the patient.

Another aspect is a method for stimulation and photobiomodulation of tissue of a patient. The method includes providing at least one lead implanted in the patient; stimulating the tissue using at least one electrode disposed on the at least one lead; and in conjunction with the stimulation, emitting light from the at least one lead using a light source to up-regulate or down-regulate at least one biomarker in the tissue.

Yet another aspect is a system for stimulation and photobiomodulation of tissue of a patient that includes at least one lead implantable in the patient, the at least one lead collectively comprising at least one electrode for delivery of electrical stimulation and at least one light emitter for emitting light for photobiomodulation; a light source; and a control module coupled to, or containing, the lead and the light source and configured for directing stimulation of the tissue using the at least one electrode disposed on the at least one lead and, in conjunction with the stimulation, directing emission of light from the at least one lead using the light source to up-regulate or down-regulate at least one biomarker in the tissue.

In at least some aspects, the at least one lead includes an optical/electrical lead including the at least one electrode and at least one light emitter. In at least some aspects, the at least one lead includes at least one electrical stimulation lead including the at least one electrode and at least one optical lead, different from the at least one electrical stimulation lead, including at least one light emitter. In at least some aspects, the biomarker includes dopamine or acetylcholine.

In at least some aspects, the at least one biomarker and determining, by a processor, whether the monitoring of the at least one biomarker meets a change condition. In at least some aspects, the method further includes, when the monitoring of the at least one biomarker meets a change condition, determining, by the processor, a change to either the stimulating of the tissue or the emitting of the light.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic side view of one embodiment of a photobiomodulation (PBM) or PBM/electrical stimulation system with a control module and a lead having a light emitter;
FIG. 2 is a schematic side view of a control module and a portion of a lead of another embodiment of a photobiomodulation (PBM) or PBM/electrical stimulation system;
FIG. 3 is a schematic side view of a portion of another embodiment of a lead with multiple light emitters;
FIG. 4 is a schematic side view of a control module and a portion of a lead of a yet another embodiment of a photobiomodulation (PBM) or PBM/electrical stimulation system;
FIG. 5 is a schematic side view of a control module, a lead extension, and a portion of a lead of a further embodiment of a photobiomodulation (PBM) or PBM/electrical stimulation system;
FIG. 6 is a block diagram of one embodiment of a PBM system;
FIG. 7 is a flowchart of one embodiment of a method for photobiomodulation;
FIG. 8 is a flowchart of one embodiment of another method for photobiomodulation;
FIG. 9 is a block diagram of another embodiment of a PBM system;
FIG. 10 is a flowchart of one embodiment of a method for delivering electrical stimulation and photobiomodulation to a patient; and
FIG. 11 is a block diagram of one embodiment of a system for PBM or PBM/electrical stimulation.

### DETAILED DESCRIPTION

The present disclosure is directed to the area of implantable photobiomodulation (PBM) or PBM/electrical stimulation systems and methods of making and using the systems. The present disclosure is also directed to implantable PBM or PBM/electrical stimulation systems that include thermal monitoring or thermal control.

The systems described herein can produce PBM or both PBM and electrical stimulation. In at least some of these embodiments, PBM can be provided through a modification of an electrical stimulation system. PBM may include, but is not necessarily limited to, optical modulation, other modulation, or other effects resulting from biological response to particular wavelengths or wavelength ranges of light or from thermal effects generated using light or from any combination thereof. It will be recognized that the thermal monitoring and thermal control arrangements and methods described herein can also be applied to implantable systems for oximetry, spectroscopy, or the like or any combination of these system and photobiomodulation systems. Unless otherwise indicated, PBM does not include photostimulation. However, photostimulation may also result during PBM.

An implantable PBM or PBM/electrical stimulation system includes at least one light source, such as a light emitting diode (LED), light emitting transistor (LET), laser diode, a vertical cavity side-emitting laser (VCSEL), an organic light emitting diode (OLED), an organic light emitting transistor (OLET), a lamp, or any other suitable light source. The light source can be used to deliver light in pulses or in a continuous wave (CW) mode, or any combination thereof.

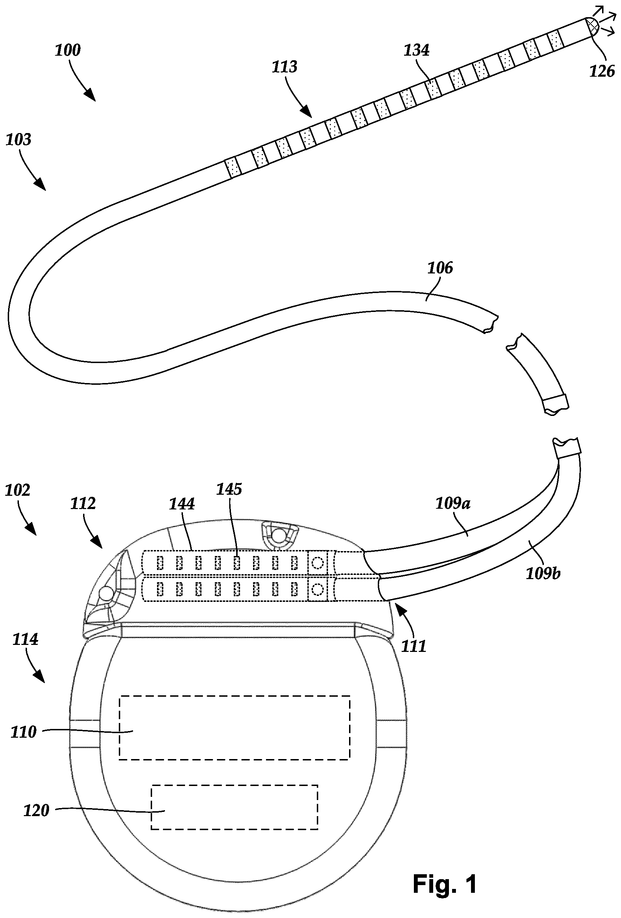

Figure 1 is a schematic side view of a portion of an embodiment of an PBM or PBM/electrical stimulation system 100 that includes a control module 102 and a lead 103. The control module includes a sealed electronics housing 114 with an electronic subassembly 110 and an optional power source 120. The control module also includes connector housing 112 (which is also often called a header) that houses a control module connector 144 that defines at least one port 111 into which a proximal end 109a, 109b of the lead 103 can be inserted. The control module connector 144 also includes connector contacts 145 disposed within each port 111. The control module 102 (or other device) can define any suitable number of ports including, for example, one, two, three, four, five, six, seven, eight, or more ports. In Figure 1, the lead 102 is shown coupled into two ports 111 defined in the control module connector 112. Other embodiments of a control module 102 may have more or fewer components.

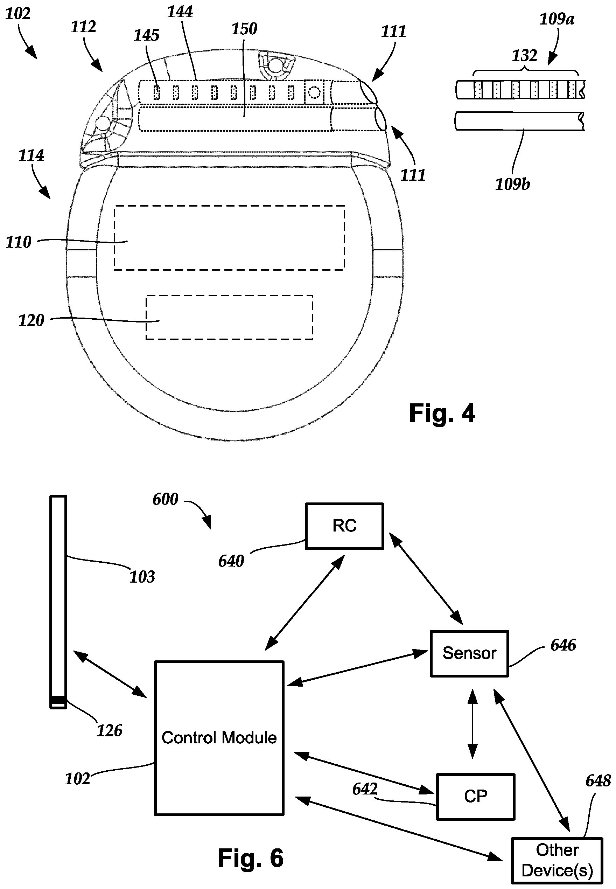

When the proximal end 109a, 109b of the lead 102 is inserted into the port 111, the connector contacts 145 can be aligned with a plurality of terminals 132 (Figure 4) disposed along the proximal end(s) of the lead. Examples of connectors in control modules are found in, for example, U.S. Patent No. 7,244,150 and 8,224,450, which are incorporated herein by reference in their entireties, as well as other references listed herein.

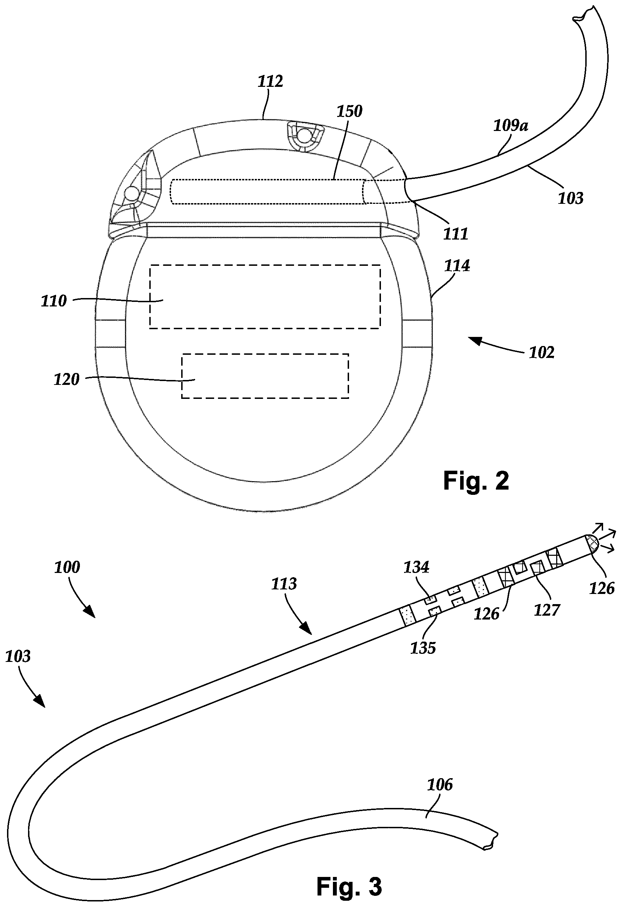

The optional power source 112 can provide power to the electronic subassembly 110. The electronic subassembly 110 is, at least in some embodiments, programmable and is configured to direct the PBM and, if present, electrical stimulation. The electronic subassembly 110 is electrically coupled to the connector contacts 144 and controls the light source 150 (Figure 2). In at least some embodiments, when the light source 150 is remote from the control module 102 (for example, disposed in the lead 103), the electronic subassembly 110 can control the light source through signals sent to the connector contacts 144 and through the terminals 168 and conductors of the lead 166 to the light source. The electrodes 134 can be ring electrodes, tip electrodes, segmented electrodes 135 (Figure 3), or any combination thereof.

The lead 103 includes a lead body 106, one or more proximal ends 109a, 109b, one or more distal ends 113, at least one light emitter 126 disposed along the distal end, one or more optional electrodes 134 disposed along the distal end, and one or more optional terminals 132 (Figure 4) disposed along the proximal end of the lead. Conductors (not shown) extend from the terminals 132 to the optional electrodes 134.

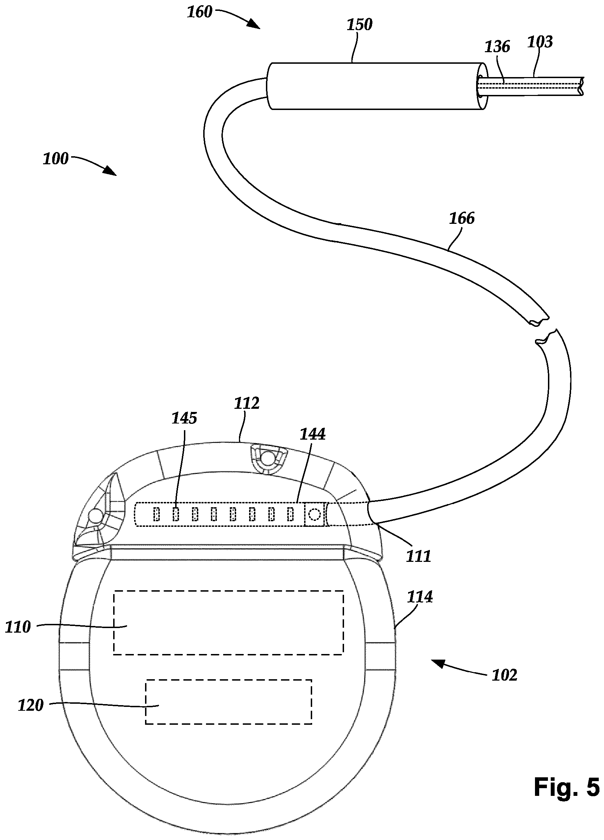

The light emitter 126 can be a light source (similar to light source 150 of Figures 2 to 4) or can be a light emission region of an optical waveguide 136 (Figure 5) or the like. When the light emitter 126 is a light source, conductors (not shown) extend from the terminals 132 (Figure 4) to the light emitter to power and operate the light source. Examples of such a light emitter can be found in U.S. Patent No. 10,335,607, incorporated herein by reference in its entirety.

When the light emitter 126 is a light emission region, the light source 250 can be disposed in the control module 102, lead 103, or other components as described below. Light from the light source is transmitted along one or more optical waveguides 136 (Figure 5) or the like to the light emission region.

Figure 2 illustrates one embodiment of a control module 102 with a light source 150 and associated optics disposed in the connector housing 112. The light source 150 provides light to a waveguide 136 (Figure 5) in the lead 103. Figure 4 illustrates another embodiment of a control module 102 and lead 103 in which one proximal end 109a of the lead 103 includes terminals 132 that couple to electrodes 134 (Figure 1) on the lead and a second proximal end 109b of the lead coupled to a light source 150 in the connector housing 112. It will be understood that, as an alternative to the embodiments in Figure 2 or Figure 4, the light source 150 can be disposed in the sealed electronics housing 114 with a waveguide extending out of the sealed electronics housing for coupling to waveguide 136 (Figure 5) in the lead 103.

Figure 5 illustrate a further embodiment of a control module 102 and a lead 102 that also includes a lead extension 160 with a lead extension body 166 that includes terminals (like the terminals 132 of Figure 4) on a proximal end for coupling to the control module connector 114 in the connector housing 112. The lead extension 160 includes a light source 150 disposed on a distal end of the lead extension and is configured to receive a proximal end of the lead 103 and deliver light into a waveguide 136 in the lead. The waveguide 136 can be a fiber optic, optical fiber, lens, or any other suitable conveyance of light. Examples of a lead extension for use with a lead with electrodes, which can be adapted to also include a light source, can be found in the references cited herein.

Although Figure 1 illustrates that light emitter 126 emitting light from a tip of the lead 102, it will be understood that a light emitter 126 can emit light from the side of the lead 102, as illustrated in Figure 3, and may emit light around the entire circumference of the lead or only a portion of the circumference as a segmented light emitter 127, as illustrated in Figure 3. Although Figure 1 illustrates a single light emitter 126, it will be understood that a lead 102 can have multiple light emitters (e.g., light sources or light emission regions), as illustrated in Figure 3, which may be the same or may differ in size, shape, orientation, emission frequency, or the like or any combination thereof.

Examples of PBM and PBM/electrical stimulation systems can be found at, for example, U.S. Patent Nos. 9,415,154; 10,335,607; and 10,814,140; U.S. Patent Applications Publications Nos. 2020/0155854; 2021/0008388; 2021/0008389; 2021/0016111; 2022/0072329; and 2022/0323781; U.S. Patent Applications Serial Nos. 18/232,621 and 18/232,649; and U.S. Patent Provisional Patent Application Serial No. 63/437,050, all of which are incorporated herein by reference in their entireties. Examples of electrical and PBM/electrical stimulation systems with leads that can be used or modified to include the elements described herein are found in, for example, U.S. Patents Nos. 6,181,969; 6,295,944; 6,391,985; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,831,742; 8,688,235; 6,175,710; 6,224,450; 6,271,094; 6,295,944; 6,364,278; and 6,391,985; U.S. Patent Applications Publication Nos. 2007/0150036; 2009/0187222; 2009/0276021; 2010/0076535; 2010/0268298; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; 2013/0105071; 2011/0005069; 2010/0268298; 2011/0130817; 2011/0130818; 2011/0078900; 2011/0238129; 2011/0313500; 2012/0016378; 2012/0046710; 2012/0165911; 2012/0197375; 2012/0203316; 2012/0203320; and 2012/0203321, all of which are incorporated herein by reference in their entireties.

PBM can be used to treat neuronal tissue to protect neurons, promote neuron survival, or promote neuronal growth or any combination thereof. Photostimulation, as used herein, is generation of a neural signal (i.e., an electrical signal) or an action potential by the activation of neural tissue (e.g., neurons or other neural cells) in response to optical irradiation. Unless otherwise indicated, PBM, as used herein, is a biological or cellular response to optical irradiation excluding photostimulation. In at least some embodiments, the biological or cellular response includes modulation (for example, up-regulation or down-regulation) of one or more chemical or biological entities. In at least some embodiments, optical stimulation using a PBM system may induce both PBM and photostimulation. In at least some embodiments, a PBM system also can be used to provide photostimulation with or without PBM.

In at least some embodiments, a PBM system can include one or more sensors to monitor one or more biomarkers (e.g., chemical or biological entities or biological signals (i.e., biosignals)) or the like. In at least some embodiments, a PBM system can monitor or measure one or more biomarkers that indicate or provide information about the protection of neurons, the promotion of neuron survival, or the promotion of neuronal growth, a change in inflammatory activity (e.g., a decrease), a change in local blood flow (e.g., an increase), or any combination thereof. In at least some embodiments, the PBM system can modify a PBM dosage in response to monitoring using, for example, sensor signals, information from the sensor(s), or information derived from the sensor signals or other monitoring or the like or any combination thereof.

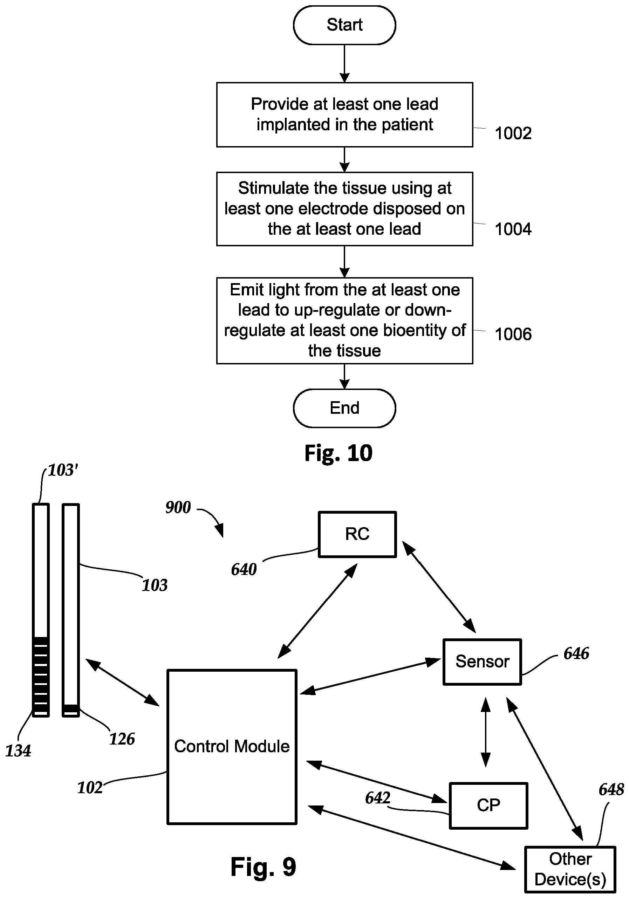

Figure 6 illustrates one embodiment of a PBM system 600 that includes at least one optical (or optical/electrical) lead 103 with one or more light emitters 126, a control module 102, at least one sensor 646, and at least one programmer or other user-operable device (for example, a clinician programmer (CP) 642, a remote control (RC) 640, or any other user-operable device(s) 648) for communication with the control module. Examples of other user-operable device(s) can include, but are not limited to, a mobile or smart phone, a laptop, a computer, a tablet, or the like. Other PBM systems can include more or fewer components. For example, in at least some embodiments, a PBM system can also include at least one electrostimulation lead 103' having one or more electrodes 134 (Figure 9).

In a PBM system 600, each sensor 646 can communicate sensor data (for example, raw sensor signals, analyzed or selected sensor signals, or information generated by analysis of the sensor signals, or the like or any combination thereof) to the control module 102, CP 642, RC 640, other user-operable device(s) 648, or any other suitable recipient, or any combination thereof. In at least some embodiments, one or more of the sensor(s) 646 can receive control signals or any other information or signals from the control module 102, CP 642, RC 640, other user-operable device(s) 648 or any combination thereof. Communication can be performed using any suitable wired or wireless methodology (for example, wired connections, WiFi, Bluetooth^{™}, infrared, near field communication, or any other communication mechanism) or any combination thereof. Communication may be direct or through one or more intermediaries (e.g., a server, the Internet, another device, or the like or any combination thereof).

In at least some embodiments, the PBM system 600 modifies optical biomodulation or dosing based on the monitoring by the sensor(s) 646. In at least some embodiments, modification of the optical biomodulation or dosing is performed without human intervention. For example, the modification can be performed by a processor of the control module 102, CP 642, RC 640, other user-operable device(s) 648, or any other suitable device or any combination thereof.

Any suitable sensor 646 can be used. In at least some embodiments, a sensor 646 can be used to measure or monitor one or more chemical (e.g., biochemical) or biological (e.g., cellular) entities, such as, for example, biochemicals (e.g., dopamine, acetylcholine (ACh), other neurotransmitters, cytokines (such as inflammatory cytokines), other proteins, glucose, oxygen, or the like), cells, cellular components, or the like. In at least some embodiments, the chemical or biological entities are found in blood, cerebrospinal fluid, other fluids, or other tissues. Other sensors 646 can be used to measure or monitor biomarkers, such as any suitable characteristic, feature, aspect, attribute, quality, or aspect of the patient or the patient's condition, disorder, disease, health, functioning or the like. Examples include, but are not limited to, motion of the patient or portion(s) of the patient (e.g., microtremor of the eye), movement (e.g., gait, rate, amount, length of time, or the like), location, sleep, exercise, balance, posture, performance of a specified task, wellness, pain, disease/disorder state, disease/disorder progression, other states of the patient, heart rate, respiration rate, blood oxygen level, blood glucose level, pulse, posture, electroencephalogram, skin temperature, core temperature, UPDRS (Unified Parkinson's Disease Rating Scape) analysis, patient imaging, or the like or any combination thereof. Examples of sensors 646 can include, but are not limited to, a chemical or biological entity sensor, an accelerometer, a gyroscope, a global positioning system (GPS) locator, a pulse oximeter, a dialysis machine or sensor, an electrical measurement device (e.g., an electroencephalography (EEG) device, an electrocardiography (ECG) device, an electrocorticography (ECoG) device, an electromyography (EMG) device, a device for obtaining evoked responses or other neural signals, or the like or any combination thereof), or the like or any combination thereof.

In at least some embodiments, at least one sensor 646 can be used to measure an amount, magnitude, or rate of change of a biomarker or a change in the amount, magnitude, or rate of change of the biomarker. In at least some embodiments, at least one sensor 646 can facilitate monitoring the effectiveness, direction, or magnitude of change (or lack of change) in neuronal survival, population, or growth over time. In at least some embodiments, the sensor(s) 646 can measure or monitor one or more proxies (e.g., metabolic function) of neuronal survival, population, or growth over time. In at least some embodiments, as an example, the sensor(s) 646 can monitor or measure neurotransmitter dynamics. In at least some embodiments, at least one of the sensor(s) 646 monitors or measures at least one biomarker indicating inhibitory, excitatory, or modulatory activity. In at least some embodiments, the location of at least one sensor is chosen such that the measurement or monitoring can reflect the effectiveness of PBM at supporting neuron survival or neural growth in a target area (such as a site related to a neurodegenerative disease).

A sensor 646 can be an implanted (or partially implanted) sensor, a worn or wearable sensor, an external sensor, or any other suitable sensor. In at least some embodiments, at least one of the sensor(s) is an implanted sensor, such as, for example, an implanted lead (for example, lead 103' of Figure 9 or a lead 103 with at least one electrode) for receiving electrical signals, an implanted chemical or optical sensor, or a sensor included as part of an implanted medical device (for example, a pacemaker, insulin pump, or the like). In at least some embodiments, at least one sensor 646 is part of the lead 103, control module 102, CP 642, RC 640, other user-operable device 648, or electrostimulation lead 103' (Figure 9). In at least some embodiments, one or more of the sensor(s) can be implanted or otherwise provide access to the same or different area of the brain in which the PBM lead(s) is/are implanted or to cerebrospinal fluid (CSF), blood, or other body fluids or tissues for monitoring of the biomarker(s). In at least some embodiments, at least one of the sensor(s) is an external sensor or a worn/wearable sensor. For example, the sensor 646 be, or be part of, a watch, a smartphone, a belt-worn sensor, a drug pump, a sensor adhesively attached to the skin of the patient, or the like. External sensors can include, for example, an chemical or biological entity analyzer or test, a camera, an EEG device, a ECG device, a ECoG device, a EMG device, or the like.

As an example, one or more sensors (e.g., a fast cyclic voltammetry sensor) can monitor dopamine in the striatum to monitor or track effectiveness of PBM at promoting neuronal survival or improved metabolic function in the substantia nigra. As another example, dopamine, acetylcholine (ACh), or other biomarkers can be monitored or measured using dialysis or microdialysis sensors or any other method for drawing or testing blood or other body fluids or tissues. In at least some embodiments, ACh can be monitored or measured for treatment of Alzheimer's disease or other diseases or disorders. In at least some embodiments, one or more inflammatory markers can be monitored or measured to track healing, for example, in cases of neural injury (e.g., trauma, stroke, or the like). In at least some embodiments, oxygen level can be monitored or measured to track progress of a PBM therapy to increase blood flow.

In at least some embodiments, monitoring or measurements by at least one sensor 646 can be performed continuously or periodically at any suitable frequency (for example, 1, 2, 5, 10, 15, or 30 seconds, 1, 2, 5, 10, 15, 20, or 30 minutes, 1, 2, 3, 4, 6, 12, or 18 hours, 1, 2, 5, or 10 days, 1, 2, 3, or 4 weeks, 1, 2, 3, 4, 6, or 12 months, or the like or any other suitable time period). Irregular periods can also be used. Different sensors 646 can have different monitoring or measurement frequencies. In at least some embodiments, monitoring or measurement by at least one sensor 646 can be performed at the request of the patient, a clinician, or any other authorized person.

In at least some embodiments, communication of signals or information from the sensor(s) 646 to the control module 102, CP 642, RC 640, other user-operable device(s) 648, or any other suitable device or any combination thereof can occur continuously or periodically at any suitable frequency (for example, 1, 2, 5, 10, 15, or 30 seconds, 1, 2, 5, 10, 15, 20, or 30 minutes, 1, 2, 3, 4, 6, 12, or 18 hours, 1, 2, 5, or 10 days, 1, 2, 3, or 4 weeks, 1, 2, 3, 4, 6, or 12 months, or the like or any other suitable time period). Irregular periods can also be used. In at least some embodiments, communication by at least one sensor 646 to the control module 102, CP 642, RC 640, other user-operable device(s) 648, or any other suitable device or any combination thereof can be performed at request of the patient, a clinician, or any other authorized person.

In at least some embodiments, the signals or information from the sensor(s) 646 can be processed by the sensor(s), control module 102, CP 642, RC 640, other user-operable device(s) 648, or any other suitable device or any combination thereof continuously or periodically at any suitable frequency (for example, 1, 2, 5, 10, 15, or 30 seconds, 1, 2, 5, 10, 15, 20, or 30 minutes, 1, 2, 3, 4, 6, 12, or 18 hours, 1, 2, 5, or 10 days, 1, 2, 3, or 4 weeks, 1, 2, 3, 4, 6, or 12 months, or the like or any other suitable time period). Irregular periods can also be used. In at least some embodiments, the signals or information from the sensor(s) can be processed by the control module 102, CP 642, RC 640, other user-operable device(s) 648, or any other suitable device or any combination thereof at the request of the patient, a clinician, or any other authorized person.

In at least some embodiments, a PBM dosage can be modified by the control module 102, CP 642, RC 640, other user-operable device(s) 648, or any other suitable device or any combination thereof in response to signals or information from the sensor(s) continuously or periodically at any suitable frequency (for example, 1, 2, 5, 10, 15, or 30 seconds, 1, 2, 5, 10, 15, 20, or 30 minutes, 1, 2, 3, 4, 6, 12, or 18 hours, 1, 2, 5, or 10 days, 1, 2, 3, or 4 weeks, 1, 2, 3, 4, 6, or 12 months, or the like or any other suitable time period). Irregular periods can also be used. In at least some embodiments, a PBM dosage can be modified by the control module 102, CP 642, RC 640, other user-operable device(s) 648, or any other suitable device or any combination thereof in response to signals or information from the sensor(s) at the request of the patient, a clinician, or any other authorized person.

PBM can be delivered continuously, periodically (at either regular or irregular intervals), or at the request of the patient, a clinician, or any other authorized person or any combination thereof. In at least some embodiments, PBM is delivered at one or more selected portions of a circadian cycle. For example, PBM may be delivered during night, day, evening, morning, or the like or any combination thereof. Such selection may be based on activities associated with the time periods or the effectiveness of the PBM or the dosage required. In at least some embodiments, PBM is delivered (or not delivered) when the patient is engaged in one or more selected activities (for example, sleeping, resting, moving, driving, eating, or the like or any combination thereof). In at least some embodiments, the PBM system may detect or otherwise determine an activity (using, for example, one or more sensors 646) or a user may indicate to the PBM system what activity is being performed, or any combination thereof. In at least some embodiments, the PBM system 600 allows a user (e.g., a patient, caregiver, or clinician) to select or modify the PBM delivery schedule and any other factors (e.g., circadian rhythm, activity, or the like) that affect PBM delivery.

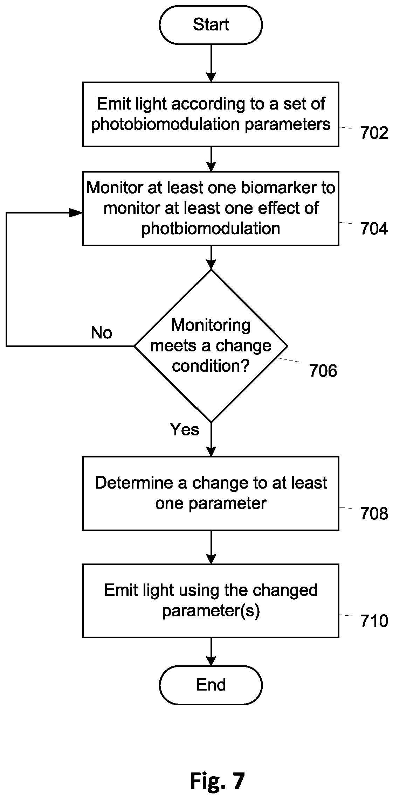

A method for photobiomodulation can include monitoring at least one biomarker and altering one or more photobiomodulation parameters in response to the monitoring, for example, altering a dosage delivered by a PBM system, such as the PBM system 600 of Figure 6 or the PBM system 900 of Figure 9. In at least some embodiments, an objective of the method includes promoting neural protection, neural survival, or neural growth or any combination thereof. Figure 7 is a flowchart of one embodiment of a method for photobiomodulation. In step 702, light is emitted from an implanted light emitter (for example, a light emitter 126 of an implanted lead 103) according to a set of photobiomodulation parameters to interact with cells or other tissue of a patient. Examples of photobiomodulation parameters include the dosage delivered by the PBM system, the amplitude or power of the emitted light, the frequency or frequencies of the emitted light, the pulse frequency (when the light is pulsed), the duration of light delivery, how often light is delivered to the patient, or the like.

In step 704, at least one biomarker is monitored (e.g., measured or observed) to, for example, monitor at least one effect (e.g., therapeutic effect or side effect) of the photobiomodulation. A biomarker can be a chemical or biological entity or a biosignal or any combination thereof. In at least some embodiments, the monitoring is performed continuously, periodically, or when requested by the user or any combination thereof. In at least some embodiments, the monitoring is performed using at least one sensor 646. Any suitable sensor(s) 646 can be used including, but not limited to, implanted, worn, wearable, or external sensors. In at least some embodiments, sensor signals, sensor measurements, analyzed sensor data, or the like is transmitted from the sensor(s) 646 to the control module 102, RC 640, CP 642, other user-operable device 648, or the like or any combination thereof. In at least some embodiments, the monitoring can be analyzed at the sensor(s) 646, control module 102, RC 640, CP 642, other user-operable device 648, or the like or any combination thereof.

In step 706, a query is made whether one or more change conditions are met by the monitoring to suggest a possible change to the PBM. Any suitable change condition(s) can be selected. Examples of change conditions include exceeding or falling below a predetermined threshold value or range corresponding to an amount, magnitude, or rate of change of a biomarker or a change in the amount, magnitude, or rate of change of the biomarker, or a relative change between two or more biomarkers, or the like or any combination thereof. As an example, a change condition may correspond to exceeding or falling below a threshold amount of dopamine, ACh, inflammatory marker(s), or oxygen or a threshold rate of change of the amount of dopamine, ACh, inflammatory marker(s), or oxygen as determined by a sensor.

In at least some embodiments, the change condition is related to, or is a proxy for, a therapeutic effect or side effect. In at least some embodiments, a user (e.g., a clinician, a programmer, a caregiver, a patient, or the like or any combination thereof) can select, define, activate, or deactivate a change condition or multiple change conditions that may be predetermined or may be defined or modified by the user. In at least some embodiments, a user (e.g., a clinician, a programmer, a caregiver, a patient, or the like or any combination thereof) can define or modify a change condition or select or modify a threshold value or range for a change condition. In at least some embodiments, the change condition can include more than one threshold value or range or may require threshold values or ranges from two or more different measurements or observations. In at least some embodiments, the change condition may also include additional factors such as, for example, a determination of an activity by the patient, a time of day, a medication status, activity level, metabolic activity level, or the like or any combination thereof. In at least some embodiments, a factor may be required for a positive determination of the change condition or may negate a positive determination of the change condition.

If no change condition is met in step 706, the procedure returns to step 704. If a change condition is met in step 706, then, in step 708, a change to at least one PBM parameter is determined based on the monitoring. For example, an intensity, amplitude, power, or frequency of the emitted light is altered to generate an increase, decrease, or other alteration in the PBM to produce a change in the response to the PBM. In step 710, light is emitted using the changed parameter(s). The method can be repeated continuously, periodically (with a regular or irregular period), when demanded by a user, or the like or any combination thereof.

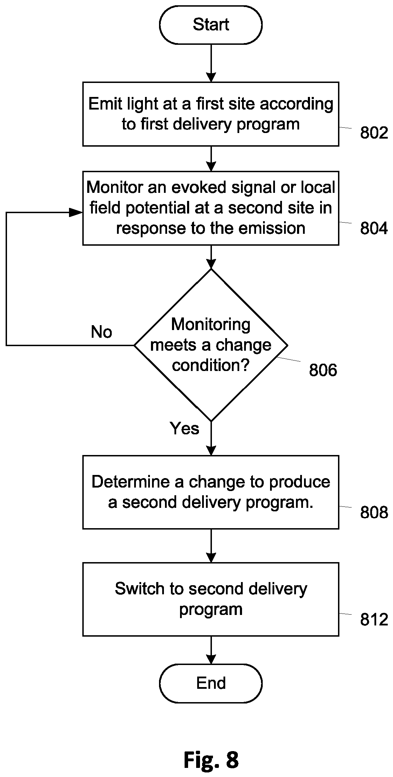

A method for photobiomodulation can include monitoring at least one biomarker and altering one or more photobiomodulation parameters, for example, altering a dosage delivered by a PBM system, such as the PBM system 600 of Figure 6 or the PBM system 900 illustrated in Figure 9. In at least some embodiments, an objective of the method includes promoting neural protection, neural survival, or neural growth or any combination thereof. Figure 8 is a flowchart of another embodiment of a method for photobiomodulation. In step 802, light is emitted from an implanted light emitter (for example, a light emitter 126 of an implanted lead 103) according to a set of photobiomodulation parameters to interact with cells or other tissue of a patient. Examples of photobiomodulation parameters include the dosage delivered by the PBM system, the amplitude or power of the emitted light, the frequency or frequencies of the emitted light, the pulse frequency (when the light is pulsed), the duration of light delivery, how often light is delivered to the patient, or the like.

In step 804, an evoked signal or a local field potential is monitored (e.g., measured or observed) at a second site (for example, a second site in the brain of the patient that is at least 5, 10, or more millimeters away from the first site in response to the light emission. As an example, a lead with a light emitter is disposed to illuminate a portion of the substantia nigra and a sensor (e.g., a lead with an electrode) is disposed to monitor an evoked signal or a local field potential at a lateral nigral target or a target that is upstream or downstream (with respect to brain signals) of the substantia nigra or any other target, such as a subgaleal target.

As another example, in the context of Parkinson's Disease (or other diseases or disorders), a first nucleus or brain area can be photobiomodulated with the response monitored or measured in a second nucleus or brain area and measure the response. A change in that response over time can reflect a change in disease condition (worsening or improving), or absolute disease state. As an example, photobiomodulating the subthalamic nucleus (STN) and monitoring or measuring the response in the globus pallidus internus (GPi) would be expected to elicit increasing amounts of activity as Parkinson's disease progresses or little or no change if the disease has been halted or slowed. As another example, photobiomodulation of the substantia nigra pars compacta (SNc) can elicit a measurable increase in dopamine, which would be reduced with disease progression or not reduced (or with lesser reduction) with successful PBM therapy.

In other embodiments, the photobiomodulation and monitoring or measuring can occur at the same site. For example, PBM of the SNc and measurement or monitoring of the evoked response of the SNc can be used to monitor disease progression. For example, as the disease progresses and cells of the SNc die, the evoked response is reduced. The evoked response not being reduced (or being reduced to a lesser extent than predicted) over time can be an indicator that the disease is not progressing and that the PBM therapy is effective.

In at least some embodiments, the light is emitted to monitor at least one effect (e.g., therapeutic effect or side effect) of the photobiomodulation. In at least some embodiments, the monitoring is performed continuously, periodically, or when requested by the user or any combination thereof. In at least some embodiments, the monitoring is performed using at least one sensor 646 such as, for example, at least one electrode 134 on an implanted lead 103. In at least some embodiments, the PBM system includes two leads 103 implanted into different portions of the brain and both including light emitter(s) 126 and electrode(s) 134. In at least some embodiments, sensor signals, sensor measurements, analyzed sensor data, or the like is transmitted from the sensor(s) 646 to the control module 102, RC 640, CP 642, other user-operable device 648, or the like or any combination thereof. The monitoring can be analyzed at the sensor(s) 646, control module 102, RC 640, CP 642, other user-operable device 648, or the like or any combination thereof.

In step 806, a query is made whether one or more change conditions are met by the monitoring to suggest a possible change to the PBM. Any suitable change condition(s) can be selected. Examples of change conditions include threshold values (e.g., above or below the threshold value) corresponding to an amount, magnitude, or rate of change of the evoked signal or local field potential or a change in the amount, magnitude, or rate of change of the evoked signal or local field potential. As an example, the monitoring can be used to measure neuronal activity over time and a change condition can be related to neuronal activity. As another example, PBM may be applied to the SNc and the local field potential monitored or measured in the cortex or STN or other part of the brain's motor circuit. A change in a beta band of the signal can indicate disease progression and lack of a change (or a smaller change than expected) can indicate PBM therapy effectiveness.

In at least some embodiments, the change condition is related to, or is a proxy for, a therapeutic effect or side effect. In at least some embodiments, a user (e.g., a clinician, a programmer, a caregiver, a patient, or the like or any combination thereof) can select, define, activate, or deactivate a change condition or multiple change conditions that may be predetermined or may be defined or modified by the user. In at least some embodiments, a user (e.g., a clinician, a programmer, a caregiver, a patient, or the like or any combination thereof) can define or modify a change condition or select or modify a threshold value or range for a change condition. In at least some embodiments, the change condition can include more than one threshold value or range or may require threshold values or ranges from two or more different measurements or observations. In at least some embodiments, the change condition may also include additional factors such as, for example, a determination of an activity by the patient, a time of day, a medication status, or the like or any combination thereof. In at least some embodiments, a factor may be required for a positive determination of the change condition or may negate a positive determination of the change condition.

If no change condition is met in step 806, the procedure returns to step 804. If a change condition is met in step 806, then, in step 808, a change to at least one PBM parameter is determined based on the monitoring. For example, an intensity, amplitude, power, or frequency of the emitted light is altered to generate an increase, decrease, or other alteration in the PBM to produce a change in the response to the PBM. In step 810, light is emitted using the changed parameter(s). The method can be repeated continuously, periodically (with a regular or irregular period), when demanded by a user, or the like or any combination thereof.

Figure 9 illustrates a PBM system 900 that includes the same components as PBM system 600 and also includes a lead 103' with one or more electrodes 134. In at least some embodiments, the lead 103 and lead 103' can be the same lead. In at least some embodiments, the PBM system 900 can be used to provide electrical stimulation in combination with photobiomodulation. In at least some embodiments, the combination of electrical stimulation and photobiomodulation provides a synergy that enhances at least one effect beyond that which is obtained individually. In at least some embodiments, the electrical stimulation and the light delivery can be coordinated temporally. In at least some embodiments, the temporal coordination provides or enhances the synergy between the electrical stimulation and the light delivery. As an example, neurons can be electrically stimulated and, during off-periods of the electrical stimulation, PBM can be provided to promote recovery after stimulation. For example, electrical stimulation can be used to promote the release of ACh in the cortex (for example, to promote memory health) and PBM can be used to promote health of the cells of the nucleus basalis of Meynert (NBM) so that these cells can continue to provide ACh. In at least some embodiments, the electrical stimulation and light delivery can occur in non-uniform patters to mimic natural variability in biological processes. In at least some embodiments, the electrical stimulation and the photobiomodulation are directed at different targets or different sites or produce different effects.

Figure 10 is a flow chart of one method for delivering electrical stimulation and photobiomodulation to a patient. In step 1002, there is at least one lead implanted in the patient. In at least some embodiments, there can be at least one electrical/optical lead that provides both electrical stimulation and light delivery. In at least some embodiments, there can be at least one electrical stimulation lead and at least one optical lead. Any other suitable arrangement of leads can be used.

In step 1004, the tissue is stimulated using at least one electrode of the electrical or electrical/optical lead. In step 1006, light is emitted from the optical or electrical/optical lead to up-regulate or down-regulate at least one bioentity (e.g., chemical or biological entity) of the target tissue. It will be understood that steps 1004 and 1006 can be performed simultaneously, partially simultaneously, or in any order. The tissue targeted for electrical stimulation can be the same tissue targeted for receiving the emitted light or can be different tissue or there can be overlap between the tissue targeted for electrical stimulation and the tissue targeted for receiving the light.

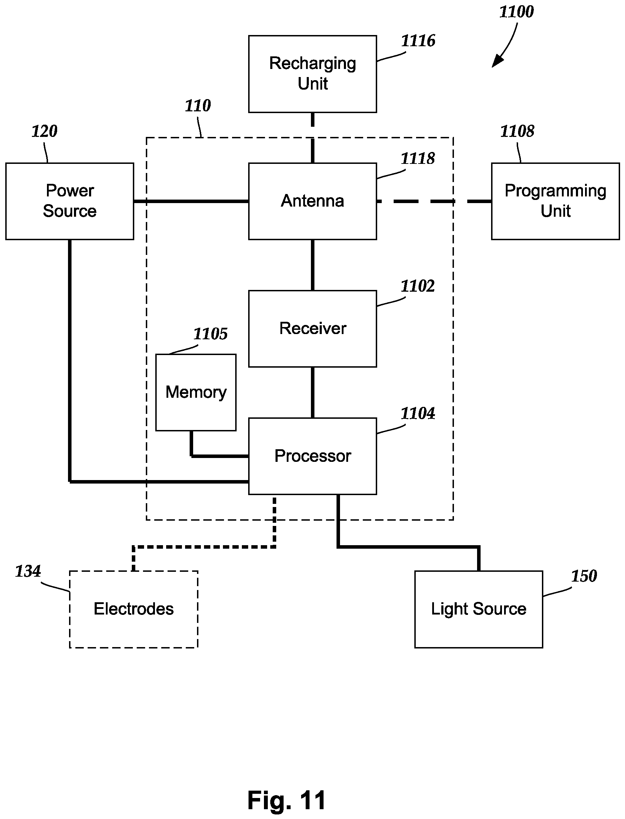

Figure 11 is a schematic overview of one embodiment of components of an PBM or PBM/electrical stimulation system 1100 including an electronic subassembly 110 disposed within a control module 102 (for example, an implantable pulse generator). It will be understood that the PBM or PBM/electrical stimulation system can include more, fewer, or different components and can have a variety of different configurations including those configurations disclosed in the stimulator references cited herein.

In at least some embodiments, selected components (for example, a power source 110, an antenna 1118, a receiver 1102, a processor 1104, and a memory 1105) of the PBM or PBM/electrical stimulation system can be positioned on one or more circuit boards or similar carriers within a sealed housing of a control module 102. Any suitable processor 1104 can be used and can be as simple as an electronic device that, for example, produces signals to direct or generate PBM or PBM/electrical stimulation at a regular interval or the processor can be capable of receiving and interpreting instructions from an external programming unit 1108 that, for example, allows modification of delivery parameters or characteristics.

The processor 1104 is generally included to control the timing and other characteristics of the PBM or PBM/electrical stimulation system. For example, the processor 1104 can, if desired, control one or more of the timing, pulse frequency, amplitude, power, or duration of the PBM or PBM/electrical stimulation. In addition, for PBM/electrical systems, in at least some embodiments, the processor 1104 can select one or more of the electrodes 134 to provide electrical stimulation, if desired, or one or more light emitters 126 to deliver light. In at least some embodiments, the processor 1104 selects which of the electrode(s) are cathodes and which electrode(s) are anodes.

Any suitable memory 1105 can be used. The memory 1105 illustrates a type of computer-readable media, namely computer-readable storage media. Computer-readable storage media may include, but is not limited to, nonvolatile, non-transitory, removable, and non-removable media implemented in any method or technology for storage of information, such as computer readable instructions, data structures, program modules, or other data. Examples of computer-readable storage media include RAM, ROM, EEPROM, flash memory, or other memory technology, magnetic storage devices, or any other medium which can be used to store the desired information and which can be accessed by a processor.

The processor 1104 is coupled to a light source 150. Any suitable light source can be used including, but not limited to, LEDs, OLEDs, LETs, OLETs, laser diodes, VCSELs, lamps, light bulbs, or the like or any combination thereof. In at least some embodiments, the PBM or PBM/electrical stimulation system may include multiple light sources. In at least some embodiments, each of the multiple light sources may emit light having a same or different wavelength or a same or different wavelength range. Any suitable wavelength or wavelength range can be used including, but not limited to, visible, near infrared, and ultraviolet wavelengths or wavelength ranges. A wavelength or wavelength range of a light source may be selected to obtain a specific therapeutic, chemical, or biological effect.

Any power source 120 can be used including, for example, a battery such as a primary battery or a rechargeable battery. Examples of other power sources include super capacitors, nuclear or atomic batteries, fuel cells, mechanical resonators, infrared collectors, flexural powered energy sources, thermally-powered energy sources, bioenergy power sources, bioelectric cells, osmotic pressure pumps, and the like. As another alternative, power can be supplied by an external power source through inductive coupling via an antenna 1118 or a secondary antenna. The external power source can be in a device that is mounted on the skin of the user or in a unit that is provided near the user on a permanent or periodic basis. In at least some embodiments, if the power source 120 is a rechargeable battery, the battery may be recharged using the antenna 1118 and a recharging unit 1116. In some embodiments, power can be provided to the battery for recharging by inductively coupling the battery to the external recharging unit 1116.

In at least some embodiments, the processor 1104 is coupled to a receiver 1102 which, in turn, is coupled to an antenna 1118. This allows the processor 1104 to receive instructions from an external source, such as programming unit 1108, to, for example, direct the delivery parameters and characteristics. The signals sent to the processor 1104 via the antenna 1118 and the receiver 1102 can be used to modify or otherwise direct the operation of the PBM or PBM/electrical stimulation system. For example, the signals may be used to modify the characteristics or delivery parameters of the PBM or PBM/electrical stimulation system. The signals may also direct the PBM or PBM/electrical stimulation system 1100 to cease operation, to start operation, to start charging the battery, or to stop charging the battery. In other embodiments, the PBM or PBM/electrical stimulation system does not include the antenna 1118 or receiver 1102 and the processor 1104 operates as initially programmed.

In at least some embodiments, the antenna 1118 is capable of receiving signals (e.g., RF signals) from an external programming unit 1108 (such as a clinician programmer or patient remote control or any other device) which can be programmed by a user, a clinician, or other individual. The programming unit 1108 can be any unit that can provide information or instructions to the PBM or PBM/electrical stimulation system 1100. In at least some embodiments, the programming unit 1108 can provide signals or information to the processor 1104 via a wireless or wired connection. One example of a suitable programming unit is a clinician programmer or other computer operated by a clinician or other user to select, set, or program delivery parameters for the PBM or PBM/electrical stimulation system. Another example of the programming unit 1108 is a remote control such as, for example, a device that is worn on the skin of the user or can be carried by the user and can have a form similar to a pager, cellular phone, or remote control, if desired. In at least some embodiments, a remote control used by a patient may have fewer options or capabilities for altering delivery parameters than a clinician programmer.

Optionally, the PBM or PBM/electrical stimulation system 1100 may include a transmitter (not shown) coupled to the processor 1104 and the antenna 1118 for transmitting signals back to the programming unit 1108 or another unit capable of receiving the signals. For example, the PBM or PBM/electrical stimulation system 1100 may transmit signals indicating whether the PBM or PBM/electrical stimulation system 1100 is operating properly or not or indicating when the battery needs to be charged or the level of charge remaining in the battery. The processor 1104 may also be capable of transmitting information about the delivery parameters or characteristics so that a user or clinician can determine or verify the delivery parameters or characteristics.

It will be understood that each block of the flowchart illustrations, and combinations of blocks in the flowchart illustrations and methods disclosed herein, can be implemented by computer program instructions. These program instructions may be provided to a processor to produce a machine, such that the instructions, which execute on the processor, create means for implementing the actions specified in the flowchart block or blocks disclosed herein. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multi-processor computer system. In addition, at least one process may also be performed concurrently with other processes, or even in a different sequence than illustrated without departing from the scope or spirit of the invention.

The computer program instructions can be stored on any suitable computer-readable medium including, but not limited to, RAM, ROM, EEPROM, flash memory or other memory technology, CD-ROM, digital versatile disks ("DVD") or other optical storage, magnetic cassettes, magnetic tape, magnetic disk storage or other magnetic storage devices, in the cloud or other non-local site, or any other medium which can be used to store the desired information and which can be accessed by a computing device.

A system can include one or more processors that can perform the methods (in whole or in part) described above. In at least some embodiments, some or all of the method may be performed using one or more non-local processor(s) (for example, processors in another device or in the cloud.) The methods, systems, and units described herein may be embodied in many different forms and should not be construed as limited to the embodiments set forth herein. Accordingly, the methods, systems, and units described herein may take the form of an entirely hardware embodiment, an entirely software embodiment or an embodiment combining software and hardware aspects. The methods described herein can be performed using any type of processor or any combination of processors where each processor performs at least part of the process. In at least some embodiments, the processor may include more than one processor.

The above specification provides a description of the manufacture and use of the invention. Since many embodiments of the invention can be made without departing from the spirit and scope of the invention, the invention also resides in the claims hereinafter appended.

## Claims

1. A photobiomodulation system, the system comprising:
a lead implantable in a patient;
a light source;
a control module coupled to, or containing, the lead and the light source and configured for directing emission of light from the lead using the light source according to a first delivery program to photobiomodulate the patient;
at least one sensor configured to monitor at least one biomarker to monitor at least one effect of the photobiomodulation; and
a processor configured for
determining whether the monitoring of the at least one biomarker meets a change condition; and
when the monitoring of the at least one biomarker meets a change condition, determining a change to the first delivery program to produce a second delivery program for emission of the light from the lead according to the second delivery program.

2. The photobiomodulation system of claim 1, wherein the change condition comprises i) a magnitude or a rate of the biomarker or ii) a magnitude or rate of a change in a magnitude of the biomarker.

3. The photobiomodulation system of any one of claims 1 or 2, wherein at least one of the at least one sensor is an implanted sensor.

4. The photobiomodulation system of any one of claims 1 to 3, wherein at least one of the at least one sensor is a worn or wearable sensor.

5. The photobiomodulation system of at least one of claims 1 to 4, wherein the biomarker comprises dopamine or acetylcholine.

6. A photobiomodulation system, the system comprising:
a lead implantable in a patient;
a light source;
a control module coupled to, or containing, the lead and the light source and configured for directing emission of light from the lead at a first site using the light source according to a first delivery program to photobiomodulate the patient;
a sensor configured to monitor an evoked signal or local field potential at a second site; and
a processor configured for
monitoring the evoked signal or local field potential at the second site in response to the emission of light;
determining whether the monitoring of the evoked signal or local field potential meets a change condition; and
when the monitoring of the evoked signal or local field potential meets a change condition, determining a change to the first delivery program to produce a second delivery program for emission of the light from the lead according to the second delivery program.

7. The photobiomodulation system of claim 6, wherein the change condition comprises i) a magnitude or change in a magnitude of the evoked signal or ii) a magnitude or a change in magnitude of a local field potential.

8. The photobiomodulation system of any one of claims 6 or 7, wherein the determining comprises determining whether the monitoring of the evoked signal or local field potential meets the change condition accounting for an additional factor selected from a circadian rhythm or a current activity of the patient.

9. The photobiomodulation system of any one of claims 1 to 8, wherein the monitoring or the determining is performed at a regular periodic interval.

10. A system for stimulation and photobiomodulation of tissue of a patient, the method comprising:
at least one lead implantable in the patient, the at least one lead collectively comprising at least one electrode for delivery of electrical stimulation and at least one light emitter for emitting light for photobiomodulation;
a light source; and
a control module coupled to, or containing, the lead and the light source and configured for directing stimulation of the tissue using the at least one electrode disposed on the at least one lead and, in conjunction with the stimulation, directing emission of light from the at least one lead using the light source to up-regulate or down-regulate at least one biomarker in the tissue.

11. The system of claim 10, wherein the at least one lead comprises an optical/electrical lead comprising the at least one electrode and at least one light emitter.

12. The system of any one of claims 10 or 11, wherein the at least one lead comprises at least one electrical stimulation lead comprising the at least one electrode and at least one optical lead, different from the at least one electrical stimulation lead, comprising at least one light emitter.

13. The system of any one of claims 10 to 12, wherein the biomarker comprises dopamine or acetylcholine.

14. The system of any one of claims 10 to 13, monitoring the at least one biomarker and determining, by a processor, whether the monitoring of the at least one biomarker meets a change condition.

15. The system of claim 14, further comprising, when the monitoring of the at least one biomarker meets a change condition, determining, by the processor, a change to either the stimulating of the tissue or the emitting of the light.
